Europäisches Patentamt

European Patent Office

Office européen des brevets

(19)

(11) Publication number: **0 293 540**
**A1**

# (12) EUROPEAN PATENT APPLICATION

(21) Application number: **87500052.3**

(22) Date of filing: **30.07.87**

(51) Int. Cl.⁴: **G01N 33/72 , //G01N33/49**

---

The title of the invention has been amended (Guidelines for Examination in the EPO, A-III, 7.3).

(30) Priority: **25.05.87 ES 8701523**

(43) Date of publication of application:
**07.12.88 Bulletin 88/49**

(84) Designated Contracting States:
**AT BE CH DE FR GB GR IT LI LU NL SE**

(71) Applicant: **BIOSYSTEMS, S.A.**
**30-4o, Costa Brava**
**Barcelona(ES)**

(72) Inventor: **Elduque Terron, Antonio**
**30-4 Costa Brava**
**Barcelona(ES)**
Inventor: **Garcia, Soler**
**30-4 Costa Brava**
**Barcelona(ES)**

(74) Representative: **Marques Alos, Fernando**
**Tuset, 34**
**E-08006 Barcelona(ES)**

---

(54) **Procedure for the separation and measurement of Hba1 glycosylated haemogiobln in blood samples.**

(57) Procedure for the separation and measurement of the percentage of HbAlc glucosylated haemoglobin in blood samples, in which a) a haemolysate is obtained from a blood sample ,b)the said haemolysate is used to impregnate a weak cationic exchange resin c)a first buffer solution is passed through the resin to effect the selective elution of the glucosylated haemoglobins HbAla and HbAlb d)a second buffer solution is passed through the resin to effect the selective elution of the HbAlc glucosylated haemoglobin and e)if required.the HbA1c content of the collected eluate is analysed and compared with the total haemoglobin present in the haemolysate -distinctive because the solution used for the selective elution of HbAlc glucosylated haemoglobin is a buffer containing ions of an alkaline metal in a concentration of between 0.02 molar and 0.05 molar and because the solution used for the prior elution of the other glucosylated haemoglobins HbA1a and HbAlb is a buffer analogous to the former, containing an organic polar reagent, soluble in aqueous solution, in a concentration of between 2% and 10% of the volume.

FIG.1

# "PROCEDURE FOR THE SEPARATION AND MEASUREMENT OF THE PERCENTAGE OF HbAlc GLUCOSYLATED HAEMOGLOBIN IN BLOOD SAMPLES"

Since the middle of this century, it has been well known that there exist different fractions of human haemoglobin and that they can be separate by physico-chemical means. However, the discovery, made by Trivelli, L.A. et al. (N. Eng. J. Med. 284(7), 353-357, 1971), that some of these fractions, known under the generic name of glucosylated haemoglobin of HbAl, come from the union of haemoglobin with different sugars and are present in varying proportions in diabetes mellitus patients, opened the way to new means of diagnosing and monitoring this disease.

For the duration of their stay in the blood, proteins, and especially haemoglobin, enter into a slow, irreversible, nonenzymatic reaction with the glucose in the blood, forming glucosylated derivatives in quantities which vary according to the average amount of glucose present during this period. In the case of haemoglobin, this time period is the same as the mean-life of the red blood cells i.e. from 4 to 6 weeks, and the reaction takes place in two main stages:

(I)Haemoglobin (HbA) + Glucose $\xrightarrow{\text{rapid}}$ pré-glucosylated haemoglobin (pre-HbAlc).

(II) pre-HbAlc $\xrightarrow{\text{slow}}$ glucosylated haemoglobin (HbAlc).

The first stage, which is reversible leads to the rapid formation of a labile, aldimine-type intermediary which is slowly transformed, in the second stage, into HbAlc glucosylated haemoglobin.

This reaction occurs in all normal individuals and, on average, HbAlc makes up approximately 5% of their haemoglobin.

In diabetic patients the presence of a greater quantity of glucose means that the reaction occurs on a wider scale and that the proportion of HbAlc is therefore higher, normally over 7%.

The measurement of glucosylated haemoglobin has been recognised, since the beginning of the 1970's as being of great value to the monitoring of diabetes mellitus patients; it informs the doctor as to the condition of the patient during the weeks preceeding the analysis, and is thus preferable to the monitoring of the glucose level at one particular time. Numerous publications have made this clear, from the beginning of the 70's to the present day. Two excellent works of reference on the subject are those by Rabhar, S. (Texas report on Biology and Medicine, 40, 373-385, 1980-81 ) and Duncan, B.B et al. (Amer.J. Epidemiol.120(2),169-189 ,1984)

The separation of the different fractions of glucosylated haemoglobin was described in 1961 by Schnek, A.G and Schroeder, W.A. (J.Amer.Chem.Soc. 83, 1472-1478, 1961) and was carried out by means of column chromatography using cellulosic or metacrylic type ion-exchange resins, impregnated with haemolysates, through which are passed buffer solutions of sodium phosphate with a pH of 6.7 at aproximately 0.049 M in sodium ion.

Since then numerous researchers and several comercial companies have adapted this method to microcolumns, permiting glucosylated haemoglobin to be measured simply and rapidly,using very small quantities of sample. All these methods are similar, using as their basic technique that described by Gabbay, K.H. et al. (J Clin. Endocrinol. Metab. 44(5), 859-864, 1977), in which the haemolysate is applied to a microcolumn filled with BioRex 70 200-400 mesh resin (a weak cationic exchange resin, a copolymer of metacrylic acid and divinylbenzene, with a particle size of between 0.045 mm and 0.075 mm) and the glucosylated haemoglobin fraction is eluted using a buffer of sodium phosphate of pH 6.7 and approximately 0.049 M in sodium ion.

The operative methods used by the various commercial companies, and likewise in the majority of published works, have the following points in common:

1. Haemolysate is obtained by treating the blood with an aqueous detergent solution and then waiting for 5-10 minutes for haemolysis to be completed.

2. The upper portion of the resin is impregnated with this haemolysate.

3. A buffer solution of sodium phosphate, the same as that recomended by Gabbay, is passed through the resin if required, to elute all the glucosylated haemoglobins.

4. A second saline solution, of greater ionic strength, can be passed through the resin, if required, to elute the remaining haemoglobins.

5. The percentage of glucosylated haemoglobins in the sample is measured by a reading, at $415_{nm}$, of the two eluted fractions or by a reading of the first fraction and of a dilution of the haemolysate .

6. During the elution process, the temperature is mantained at a constant value, usually between 20° C and 25° C.

All of these methods, although widely used in clinical diagnosis, have two important disadvantages:

a) They are highly sensitive to the conditions under which the experiment is performed, and especially to variations in temperature.

b) They permit only an overall quantification of the glucosylated haemoglobins; as this group contains, as well as HbAlc, the other haemoglobins HbAla and HbAlb derived from the union of haemoglobin with sugars other than glucose, this can lead to problems in the interpretation of the results.

The problem of temperature dependency has been partially solved by using alternative techniques such as affinity chromatography, colorimetry, electrophoresis and isoelectric focusing. All these techniques, although solving the temperature problem, present other difficulties of various kinds which have meant that their use in routine analysis is not as widespread as that of ion exchange chromatography.

The problem of the elution of all the glucosylated haemoglobins together has led various researchers to develop other methods, one of which has been commercialized by the firm BioRad Laboratories for the specific measurement of HbAlc. All of these other methods are derived from the method described earlier for the glucosylated haemoglobins and are based fundamentally upon the use of a first eluant, of a lower ionic strength than for the elution of HbAl, with which it is possible to elute, firstly, the HbAla and HbAlb haemoglobins; a second eluant, of greater ionic strength elutes the HbAlc, leaving the non-glucosylated haemoglobins in the resin.

Although the use of different ionic strengths is useful as a method of achieving separation, it is not entirely effective since, on the one hand, it is necessary to work under smooth conditions which slow down the descent of the peaks and make the process longer and on the other, this method produces a considerable diffusion of the bands as they pass through the resin; as a result, the entrainment tails of the peaks are considerable and consequently there is a high degree of sensitivity to small variations in the pH, ionic strength, temperature or length of the resin.

Schifreen, R.S. et al. (Clin. Chem. 26(3), 466-472, 1980), working with Bio Rex 70 resin in high performance liquid chromatography (HPLC), i.e. with a single column which is re-equilibrated between samples, describe how the addition of ethanol to the buffer used for the mobile phase aids the rapid re-equilibration of the resin, since this alcohol reduces the pKa of the resin's ionizable groups. Betton, D. et al (Ann. Biol. Clin. 38, 375-377, 1980) has exploited this reduction in pKa, adding the ethanol to the buffer used in a commercial kit, to achieve an improved resolution of the technique and thus to obtain the separation of HbAlc. However, the usefulness of the direct application of these conditions in routine analysis is doubtful; firstly, as the researchers themselves indicate, it lengthens the process by 30 minutes; secondly, the entrainment of the HbAlc peak is very considerable and makes it difficult to separate it completely from the fraction of non-glucosylated haemoglobins and finally, the reagents contain potassium cyanide which is highly toxic.

This invention provides an accurate, reliable and repeatable method for the routine measurement of the percentage of HbA1c in blood samples and is thus highly suited to the diagnosis and monitoring of diabetes mellitus patients. In contrast to the methods presently in existence, which permit either the quantification of all the glucosylated haemoglobins together or else are selective for HbAlc but produce an unsatisfactory separation of the chromatographic bands, this method permits the selective measurement of HbAlc with an excellent separation of the different peaks using a very small working volume; it thus offers a greater accuracy, reliability and repeatability than existing chromatographic methods.

The method requires a correct conjunction of three basic parameters: the acidity of the resin and of the eluant buffer solutions. the concentration of alkaline ions in the eluant buffer solutions and the addition of an organic polar reagent to these solutions.

The working pH is fundamental to chromatographic separation as it directly affects the degree of dissociation of the ionizable groups both of the resin and of the haemoglobins; it is specially important in this technique which is extraordinarily sensitive to variations in pH. The pH must be exact to the hundredth, at minimum, in order to obtain repeatable conditions. It is normally advisable to work at sufficiently high pH values to allow the ionizable groups of the resin to dissociate and thus to retain the cations; but the pH should not be very much higher than the pKa of the ionizable groups of the glucosylated haemoglobin so that this maintains its positive charge and is thus retained by the column.

The majority of the methods described, including the commercial methods, use buffer solutions of pH 6.70 as eluants, this being the pH value employed in the above-mentioned Schnek and Schroeder method for the separation of the haemoglobin fractions. With this pH value, a considerable retention of HbAlc is obtained which would be sufficient for non-routine works of research or monitoring but which is not quite sufficient for it to be of use in routine clinical analysis since, in order to prevent excessive retention which greatly lengthens the process and makes it of little practical use in clinical diagnosis, it is necessary, to achieve elution of HbAlc to use a buffer solution containing alkaline ions, basically sodium ions, in concentrations higher than 0.06 M. These ionic strength values cause a not very specific disunion of the resin and the haemoglobins (including the non-glucosylated haemoglobins) and therefore the widening of the bands and the start of the descent of the non-glucosylated haemoblogin band.

The method proposed in this invention uses a working pH value higher than that normally employed, of over 7.0. This pH value leads to a rapid elution of all the glucosylated haemoglobins, HbAlc as well as HbAla and HbAlb, even under conditions of low ionic strength (below 0.04 M in alkaline ions) which permits a much better retention of the non-glucosylated haemoglobins by the resin and a better focusing of the glucosylated haemoglobin peaks.

The prior elution of the fraction containing the other glucosylated haemoglobins, i.e. HbAla and HbAlb, is achieved by passing through the resin a buffer solution derived from that described earlier and to which has been added an organic polar reagent (ethylic alcohol for example) in sufficient quantities (normally between 2% and 10%) to retard the elution of HbAlc without preventing elution of the other two glucosylated haemoglobins, making use in this way of the above-mentioned effect, described by Schifreen, upon the pKa of the resin's ionizable groups. In principle, for the purposes of the proposed method, any organic polar reagent can be used that has enough polar groups for it to be soluble in aqueous buffer solutions, and enough non-polar groups in order to modify the pKa of the resin's ionizable groups, like for example short-chain aliphatic alcohols.

The remaining stages of the process, i.e. the preparation of the sample, the optional elution of the nonglucosylated haemoglobins and the reading and calculation of the results, are carried out following the procedures described below:

1. Preparation of the sample: -Three basic conditions are necessary to the preparation of the sample prior to its application to the resin:

a) Breakage of the membrane of the red blood cells with the liberation of their content, particularly haemoglobin.

b) Elimination of the labile pre -HbAlc fraction (Aldimine) which could produce a falsification due to short term variations.

c) Suitability of the haemolysate to the working pH. The three conditions are obtained using a buffer of 35mM sodium phosphate of a pH of around 5.0 containing Triton X-100 in a concentration of approximately 0.5%. Firstly, the presence of the surface-active agent causes the dissolution of the membrane and the liberation of haemoglobin; secondly, the moderate acidity of the medium causes the dissociation of the aldimine which reverts to free haemoglobin and glucose; finally, the application to the medium of the buffer gives the sample a pH value which is suitable for the subsequent chromatography.

2. Optional elution of the non-glucosylated haemoglobins: Having eluted the HbAlc fraction, the fraction of non-glucosylated haemoglobins remaining in the column can also be eluted if required, by means of the passage of a solution of high ionic strength, for example 1M sodium chloride. The fraction thus collected is used, together with those previously collected, to calculate the percentage of HbAlc. Alternatively, it is possible not to elute this fraction and to calculate the percentage by means of a direct comparison with the total quantity of haemoglobin present in the haemolysate which is applied to the column.

3. Reading and calculation of the results: The measurement of the quantity of haemoglobin in the different eluted fractions, or in the haemolysate, is carried out by a direct photometric reading at 415 nanometres of the optic density of the different fractions (using dilutions of the eluted samples or of the haemolysate, as the case may be).

The calculation of the concentration of HbAlc (expressed as a percentage of the total haemoglobin) is carried out by dividing the O.D. value corresponding to this fraction by the O.D. value of the haemolysate (corrected according to volumes and dilutions) or by adding the (corrected) O.D. values of all the eluted fractions, as the case may be.

In order that the method proposed in this invention be better understood, it can be described as follows:

a) Preparation of the haemolysate and elimination of the labile fraction: The blood is mixed with the haemolyzing reagent and the mixture is incubated at 37°C for 20 minutes.

b) Application to the column: The upper portion of the resin is impregnated with the haemolysate thus obtained; the column wall is then washed with a small quantity of a buffer solution which is used also in the next stage.

c) Elution of the HbAlc fraction: A first buffer solution is passed through the resin which causes the elution of the fraction containing the other glucosylated haemoglobins HbAla and HbAlb which is discarded. Once this solution has passed completely through the resin, a second buffer t solution is added which causes the elution of the HbAlc fraction which is collected.

d) Preparation of the total haemoglobin sample: One part of the haemolysate obtained in stage a) is diluted with buffer and distilled water in a proportion of 1:240 aproximately.

4

e) Reading and calculation: The percentage of HbAlc in the sample is obtained by a photometric reading at 415 nm of the HbAlc fraction and of the total haemoglobin sample and by then dividing the O.D. value of the former by that of the latter (corrected as appropriate according to their dilution).

Two purely illustrative, non-restrictive examples of the practical possibilities for the execution of this method are set out below, as an integral part of this descriptive paper, to provide a fuller explanation of the proposal.

## EXAMPLE I

This example describes in detail the execution of the complete method and its application to the calculation of the percentage of HbAlc in a series of samples taken from normal individuals and from diabetic patients and shows that the method is capable of distinguishing between these population groups.

### 1. Preparation of the columns.

The resin, of the Amberlite CG-50 type which is a weak cationic exchange resin, copolymer of metacrylic acid and divinylbenzene, with a particle size of between 200 and 400 mesh, is equilibrated with a buffer solution of phosphates of pH 7.0, containing sodium ion in a-concentration approximately equal to 0.04 M, until the conductivity and pH of the eluates of a microcolumn full of the resin are the same as those of the buffer solution.

Plastic columns of approximately 65 mm long and 8 mm in diameter are filled with the resin suspension, introducing aproximately 0.5 g of resin (dry weight) into each column so that the bed of the resin, once sedimented, is approximately 40 mm high. Discs of porous polyethylene, 1 mm thick, are placed at the upper and lower ends of the column; the lower disc supports the resin and the upper disc permits a perfectly homogeneous application of the sample.

### 2. Preparation of the sample.

0,05 mL of blood are mixed with 0.2 mL of 0.035 M sodium phosphate buffer of pH 5.0, containing approximately 0.5% Triton X-100 surface-active agent. The mixture is stirred (vortex) and is then incubated at 37° C for 20 minutes. Once the incubation time is completed, the haemolysate is left to regain normal temperature before being applied.

### 3. Application of the sample to the column.

Firstly, both ends of the column are opened to let the liquid they contain flow out entirely; this liquid is then discarded. 0.05 mL of the haemolysate are then carefully placed upon the upper filter which has previously been adjusted, with a rod, to the level of the resin bed. Once the haemolysate has completely penetrated the resin, impregnating it, 0.02 mL of the buffer solution of 0.015 M sodium phosphate of pH 7.0, containing approximately 0.039 M of sodium ions and to which has previously been added 5% of ethylic alcohol, are added to the column in order to wash out the remains of the application from the column wall and begin the elution. The eluate is discarded. Both in this phase and in the following, the temperature of the columns and of the reagents is held constant at 23± 0.2° C if necessary, they can be placed into a thermostatic bath.

### 4. Elution of the glucosylated haemoglobins.

Once the column has stopped dripping, 2.0 mL of buffer of 0.015 M phosphates of pH 7.0 containing 0.039 M in sodium ions and to which has been added 5% ethylic alcohol, are added to the column. The eluate is collected. It contains the fraction of the other glucosylated haemoglobins HbAla and HbAlb. (This fraction is collected only for the purposes of this example, whereas in routine analysis its collection is not necessary). Next, 4.0 mL of buffer of 0.015 M phosphates of pH 7.0 containing 0.039 M in sodium ions are added to the column, once it has stopped dripping. The eluate, containing HbAlc, is collected.

5. Preparation of the total haemoglobin sample.

0.05 mL of the haemolysate collected in step 2 are mixed with 4.0 mL of 1 M sodium chloride buffer and 8.0 mL of distilled water. It is stirred vigorously (vortex) until a homogenous mixture is obtained.

6. Reading and calculation of the results.

Using a spectrophotometer, or, as the case may be, a photometer equipped with an appropriate filter, the. O.D. (optic density) of the two eluates collected in step 4 (O.D. (HbAla + b) and O.D. (HbAlc))and of the sample prepared in step 5 (0.D$_{TOTAL}$Hb) are read at 415 nm. The percentages of the two fractions are calculated as follows:

$$\% \ HbAla + b = \frac{O.D. \ (HbAla + b)}{6 \ X \ O.D. \ (_{TOTAL}Hb)} \ X \ 100$$

$$\% \ HbAlc = \frac{O.D. \ (HbAlc)}{3 \ X \ O.D. \ (_{TOTAL}Hb)} \ X \ 100$$

The technique was carried out working with two groups of 20 samples each, the first taken from normal patients and the second from persons classified as diabetics, without any special restrictions as to the state of the illness. The values obtained appear in the following table:

6

| | | NORMAL | | | DIABETIC | |
|---|---|---|---|---|---|---|
| Sample | %HbA1a+b | %HbA1c | | Sample | %HbA1a+c | %HbA1c |
| 1 | 2.2 | 5.2 | | 21 | 3.1 | 10.1 |
| 2 | 2.3 | 5.3 | | 22 | 3.4 | 9.5 |
| 3 | 2.4 | 5.3 | | 23 | 3.8 | 11.3 |
| 4 | 2.3 | 5.8 | | 24 | 3.1 | 9.6 |
| 5 | 2.2 | 5.5 | | 25 | 2.9 | 8.4 |
| 6 | 2.1 | 5.0 | | 26 | 3.3 | 9.7 |
| 7 | 2.2 | 5.1 | | 27 | 2.9 | 9.3 |
| 8 | 2.4 | 4.9 | | 28 | 2.3 | 7.2 |
| 9 | 2.1 | 5.4 | | 29 | 2.9 | 8.9 |
| 10 | 2.3 | 4.6 | | 30 | 3.4 | 12.2 |
| 11 | 2.0 | 5.4 | | 31 | 3.8 | 13.0 |
| 12 | 2.0 | 5.0 | | 32 | 2.9 | 7.3 |
| 13 | 2.0 | 4.9 | | 33 | 2.8 | 10.2 |
| 14 | 2.0 | 4.7 | | 34 | 3.1 | 9.4 |
| 15 | 2.2 | 4.6 | | 35 | 5.4 | 17.1 |
| 16 | 2.3 | 5.2 | | 36 | 4.5 | 10.3 |
| 17 | 1.9 | 4.9 | | 37 | 3.4 | 8.8 |
| 18 | 2.2 | 5.1 | | 38 | 3.2 | 9.6 |
| 19 | 2.8 | 5.6 | | 39 | 3.4 | 10.4 |
| 20 | 2.2 | 5.3 | | 40 | 3.5 | 10.4 |

The average values obtained were:

Normal samples : % HbA1a+b = 2.21 ± 0.20%

% HbAlc = 5.14 ± 0.32%

Diabetic samples : % HbA1a+b = 3.36 ± 0.66%

% HbAlc = 10.14 ± 2.15%

It can be observed, on the one hand, that with the % HbAlc values it is possible to distinguish perfectly between the two population groups as the average value of the diabetic group is 97% higher than that of the normal group with relatively narrow margins of variation. On the other hand, it can be observed that this increase in the percentage of HbAlc is more pronounced than the increase in HbAla + b, which is, only 52%; this confirms the particular usefulness of the analysis of HbAlc compared to an analysis of all the glucosylated haemoglobins together.

EXAMPLE II

This example describes in detail the separation of the peaks of glucosylated haemoglobins, examining the elution profile of the chromatograph and comparing it with those obtained with two other methods presently in use.

As well as an examination of the elution profiles, calculations were made, to provide descriptive parameters for the resolution of the chromatography, of the width of the peaks at mid height, the depth of the troughs compared with that of the HbAlc peak, the distance travelled down the column by the front of the non-glucosylated band at the end of the HbAlc elution and the distance between the front and the end of the column, also at the end of the elution process.

The operative method described in the preceeding example was used, although the eluates of the two glucosylated fractions wre collected in separate fractions of 0.5 mL, whose O.D. values were read at 415 nm.

The other two methods were carried out following the instructions strictly except in the collection of the eluates; this was carried out in the same way as described for the invention. In order to identify these methods, for the purposes of this example, the older method, which uses a greater resin length, a larger quantity of sample and greater volumes, will be referred to as the "macro" method and another, analogous method, recently developed, using smaller quantities of resin, sample and buffer solutions, will be referred to as the "micro" method.

In each of the three cases a normal sample and a diabetic sample were used. The elution profiles were obtained by plotting the O.D. values of each fraction at 415 nm against the corresponding elution volumes, expressed in millilitres (mL),

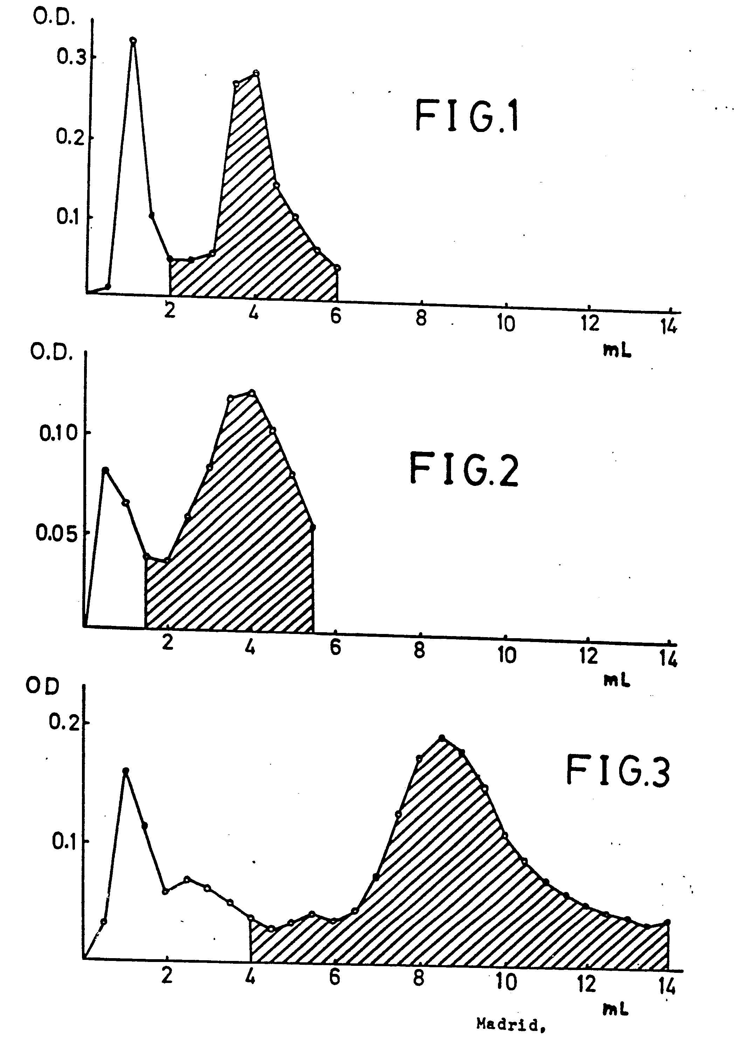

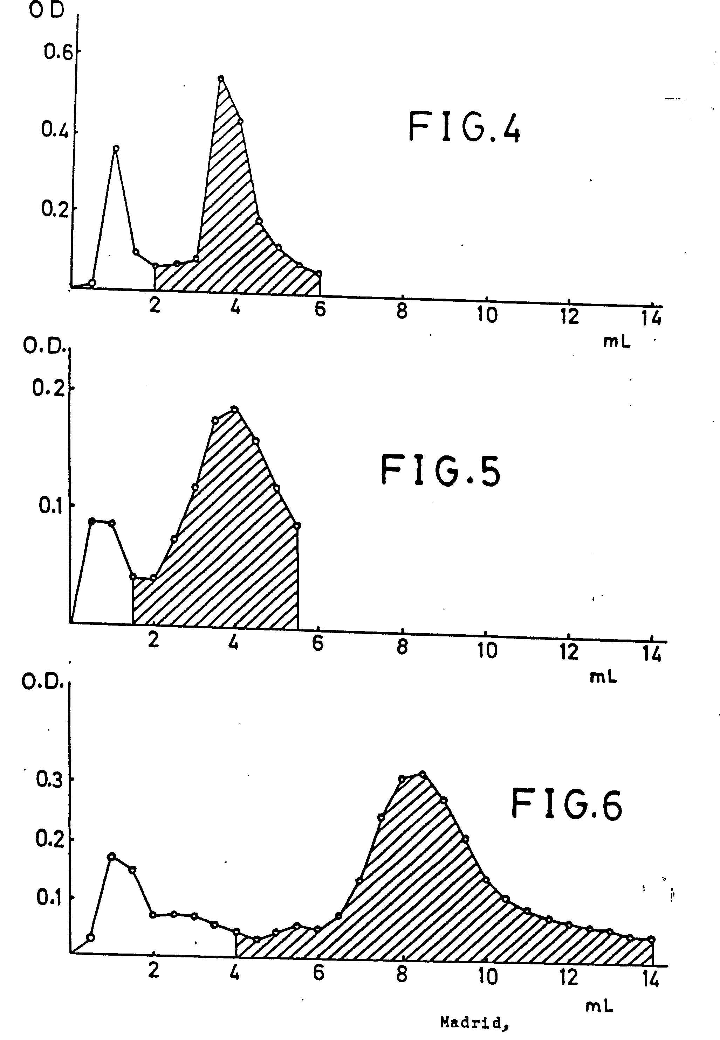

For a further clarification of this example, two sheets are annexed to this paper showing the graphs obtained from these analyses. Figure 1 shows the analysis of a normal sample with the proposed method, figure 2 shows a normal sample analysed using the "micro" method, figure 3 a normal sample with the "macro" method, figure 4 a diabetic sample analysed with the proposed method, figure 5 a diabetic sample with the "micro" method, and figure 6 a diabetic sample analysed by means of the "macro" method. The shaded areas show the fraction which is collected, in each case, as the HbAlc peak.

The table below shows the following characteristic parametres of the chromatograph:
- O.D. of the first fraction collected in the HbAlc peak, expressed as a percentage of the maximun (H1).
- O.D. of the last fraction collected in the HbAlc peak, expressed as a percentage of the maximun (H2).
- Width of the HbA1a + b peak at mid height, expressed in mL (D1).
- Width of the HbAlc peak at mid height, expressed in mL (D2).
- Distance travelled down the resin by the HbAO front, once the elution of HbAlc is completed, expressed in mm (F).
- Distance between the HbAO front and the end of the column, once the elution of HbAlc is completed, expressed in mm (L).

(The parametres are similar for the normal sample and for the diabetic sample in the three methods, and are expressed as averages).

|  | Proposed Method | "Micro" Method | "Macro" Method |
|---|---|---|---|
| H1 | 14 | 26 | 12 |
| H2 | 13 | 46 | 18 |
| D1 | 0.43 | 1.28 | 1.10 |
| D2 | 1.03 | 2.65 | 2.95 |
| F | 10 | 10 | 15 |
| L | 30 | 8 | 10 |

From the point of view of the chromatographic profile, the results which, as can be observed, are similar in each case for the normal sample and the diabetic sample, make the following points quite clear:

1. As regards the speed of the test, of great importance from the clinical point of view, the method proposed in this invention and the so-called "micro" method are comparable (6 mL and 5.5 mL in total, respectively, for the collection of the HbAlc) and greatly superior to the so-called "macro" method which requires 14 mL to achieve the same end.

2. As regards the separation of the HbAlc peak, the height of its first and last points in relation to the maximun has been chosen as a representative parameter. It can be observed both in the graphs and in the table that the proposed method is comparable to the "macro" method (the points are approximately 15% of the maximum) and that both are greatly superior to the "micro" method, especially as regards the last point, which in this case represents 46% of the maximum.

3. As regards the width of the eluated peaks, which has been expressed, as is usual in chromatography, at mid height, the proposed method can be observed to be greatly superior to the other two methods in both peaks; this can be deduced from the data in the table (D1 and D2) and from the graph where considerable differences can easily be observed in the width of the bands.

4. As regards the retention of the band of non-glucosylated haemoglobins (HbAO) and the risk that this band may begin its elution before that of the HbAlc is completed, causing serious interference, the proposed method shows itself to be greatly superior to the other two methods; whilst the band retention is comparable to that of the "micro" method (the front travels 10 mm) and superior to that of the "macro" method (the front travels 15 mm), its separation from the end of the HbAlc band is very much better (due to the length of the column - 30 mm as compared to 8 mm and 10 mm), especially compared to the "micro" method which, to quicken the process, uses a very short column where the small separation (8 mm) seems to lead to a premature cut of the HbAlc peak, as can be observed in the graphs.

It can thus be concluded that this invention offers a method which is much quicker than the traditional method and as quick as the other, recently developed method whilst being much more resolutive and reliable than both.

The proposed method, therefore, shows a rapidity, resolution and accuracy which are clearly superior, as a whole, to those of the methods presently in use; it is thus highly suited for use as a rapid, reliable and repeatable method for routine analysis of HbAlc.

Having completed the description with which this paper is concerned, it should be stressed that variations are possible in the details of execution of the idea here put forward; that is to say that they can undergo small modifications based always upon the fundamental principles of the idea which are essentially those expressed in the above paragraphs of the description.

The Patents Law presently in force states that changes in form, dimension, proportion or materials of an object already under patent do not qualify for patenting; the legislating criteria are thus established, ensuring that once an idea having a pratical and industrializable application has been patented, it cannot be presented as a new and original idea on the basis of slight modifications having been introduced.

## Claims

1. THE PROCEDURE FOR THE SEPARATION AND MEASUREMENT OF THE PERCENTAGE OF HbAlc GLUCOSYLATED HAEMOGLOBIN IN BLOOD SAMPLES in which a) a haemolysate is obtained from a blood sample, b) this haemolysate is used to impregnate a weak cationic exchange resin, c) a first buffer solution is passed through the resin to effect the selective elution of the other glucosylated haemoglobins HbAla and HbAlb, d) a second buffer solution is passed through the resin to effect the selective elution of HbAlc glucosylated haemoglobin and e) the HbAlc content of the collected eluate is analysed, if required, and compared with the total haemoglobin present in the haemolysate, is distinctive because the solution used fot the selective elution of HbAlc glucosylated haemoglobin is a buffer containing ions of an alkaline metal in a concentration of between 0.02 molar and 0.05 molar and because the solution used for the prior elution of the other glucosylated haemoglobins HbAla and HbAlb is a buffer analogous to the former containing an organic polar reagent, soluble in aqueous solution, in a concentration of between 2% and 10% of the volume.

2. THE PROCEDURE FOR THE SEPARATION AND MEASUREMENT OF THE PERCENTAGE OF HbAlc GLUCOSYLATED HAEMOGLOBIN IN BLOOD SAMPLES, in accordance with Claim 1, is distinctive because the organic polar reagent is a short-chain aliphatic alcohol.

3. THE PROCEDURE FOR THE SEPARATION AND MEASUREMENT OF THE PERCENTAGE OF HbAlc GLUCOSYLATED HAEMOGLOBIN IN BLOOD SAMPLES, in accordance with Claim 2, is distinctive because the alcohol used is ethylic alcohol.

4. THE PROCEDURE FOR THE SEPARATION AND MEASUREMENT OF THE PERCENTAGE OF HbAlc GLUCOSYLATED HAEMOGLOBIN IN BLOOD SAMPLES, in accordance with Claims 1 to 3, is distinctive because the buffers used in the elutions are of sodium phosphate at pH values of between 6.7 and 7.2.

5. THE PROCEDURE FOR THE SEPARATION AND MEASUREMENT OF THE PERCENTAGE OF HbAlc GLUCOSYLATED HAEMOGLOBIN IN BLOOD SAMPLES, in accordance with Claim 4, is distinctive because the concentration of sodium ions is the same in both buffers and is of approximately 0.04 molar.

6. THE PROCEDURE FOR THE SEPARATION AND MEASUREMENT OF THE PERCENTAGE OF HbAlc GLUCOSYLATED HAEMOGLOBIN IN BLOOD SAMPLES, in accordance with Claim 3, is distinctive because the concentration of ethylic alcohol is of between 3% and 7%.

7. THE PROCEDURE FOR THE SEPARATION AND MEASUREMENT OF THE PERCENTAGE OF HbAlc GLUCOSYLATED HAEMOGLOBIN IN BLOOD SAMPLES, in accordance with Claims 1 to 6, is distinctive because the haemolyzing reagent is a buffer t of 35 mM sodium phosphate of pH 5.0, containing 0.5% surface-active agent.

8. THE PROCEDURE FOR THE SEPARATION AND MEASUREMENT OF THE PERCENTAGE OF HbAlc GLUCOSYLATED HAEMOGLOBIN IN BLOOD SAMPLES, in accordance with Claim 7, is distinctive because the surface-active agent used is Triton X-100.

9. THE PROCEDURE FOR THE SEPARATION AND MEASUREMENT OF THE PERCENTAGE OF HbAlc GLUCOSYLATED HAEMOGLOBIN IN BLOOD SAMPLES, in accordance with Claims 1 to 8, is distinctive because the height of the resin in the column is of between 35 mm and 45 mm.

10. THE EQUIPMENT USED TO CARRY OUT THE PROCEDURE is distinctive in essence because it comprises:
- Columns with exchange resin.
- A buffer solution containing ions of an alkaline metal in a concentration of between 0.02 molar and 0.05 molar and an organic polar reagent soluble in aqueous solution in a concentration of between 2% and 10%.
- A buffer solution containing ions of an alkaline metal in a concentration of between 0.02 molar and 0.05 molar.

11. THE EQUIPMENT USED TO CARRY OUT THE PROCEDURE is distinctive in essence because it also contains an aqueous solution of surface-active agent to be used as haemolyzer.

12. THE PROCEDURE FOR THE SEPARATION AND MEASUREMENT OF THE PERCENTAGE OF HbAlc GLUCOSYLATED HAEMOGLOBIN IN BLOOD SAMPLES.

FIG.1

FIG.2

FIG.3

Madrid,

BIOSYSTEMS, S.A.

FIG.4

FIG.5

FIG.6

Madrid,

European Patent Office

**PARTIAL EUROPEAN SEARCH REPORT**
which under Rule 45 of the European Patent Convention
shall be considered, for the purposes of subsequent
proceedings, as the European search report

Application number

EP 87 500 052.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | <u>US - A - 4 389 491</u> (M.S. HANAMOTO et al.)<br>* column 5, line 45 - column 6, line 31; claims 1-32 * | 1 | G 01 N 33/72<br>// G 01 N 33/49 |
| A | | 4,5,7-11 | |
| A,D | CLINICAL CHEMISTRY, vol. 26, no. 3, 1980, pages 466-472, R.S. SCHIFREEN et al.: "Accuracy, precision, and stability in measurement of hemoglobin A1c by "high-performance" cation-exchange chromatography"<br>* pages 466-470 * | 1-4,6,10,11 | |
| A | <u>US - A - 4 436 820</u> (P.C. REITER)<br>* column 3, lines 40-46; claims 1, 8-10,12-15 * | 1,4,7,8,10,11 | TECHNICAL FIELDS SEARCHED (Int. Cl.4)<br><br>G 01 N 33/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-11
Claims searched incompletely: —
Claims not searched: 12
Reason for the limitation of the search:

Formulation is too broad

| Place of search | Date of completion of the search | Examiner |
|---|---|---|
| BERLIN | 12-07-1988 | DE KOK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

| | DOCUMENTS CONSIDERED TO BE RELEVANT | | CLASSIFICATION OF THE APPLICATION (Int. Cl.⁴) |
|---|---|---|---|
| Category | Citation of document with indication, where appropriate, of relevant passages | Relevant to claim | |
| A | CHEMICAL ABSTRACTS, vol. 91, no. 3, 16th July 1979, page 272, column 2, abstract no. 16055m; J. JONGENEEL et al.: "Optimizing measurement of glycosylated hemoglobins", & CLIN. CHEM. 1979, 25(4), 642 ---- | 1,4,10 | |

TECHNICAL FIELDS SEARCHED (Int. Cl.⁴)

EPO Form 1505.3   06.78